# EUROPÄISCHE PATENTSCHRIFT

(11) **EP 0 355 726 B1**
(45) Veröffentlichungstag und Bekanntmachung des Hinweises auf die Patenterteilung: **07.01.1993**
(21) Anmeldenummer: 89115237.3
(22) Anmeldetag: 18.08.1989
(51) Int. Cl.: C25B 3/02, C25B 3/10, C07C 43/12, C07C 59/135

(54) **Verfahren zur elektrolytischen Decarboxylierung einer Perfluorcarbonsäure oder deren löslichen Salzen und anschliessende Dimerisierung der dabei entstehenden Radikale**
Method for the electrolytic decarboxylation of a perfluorocarboxylic acid or soluble salts thereof, and subsequent dimerisation of the radicals so formed
Procédé pour la décarboxylation électrolytique d'un acide perfluorocarboxylique ou de leurs sels solubles et une dimérisation consécutive ds radicaux ainsi obtenus

(30) Priorität: 25.08.1988 DE 3828848
(43) Veröffentlichungstag der Anmeldung: 28.02.1990
(73) Patentinhaber: HOECHST AKTIENGESELLSCHAFT, 65926 Frankfurt am Main (DE)
(72) Erfinder: Blickle, Peter, Dr., D-6233 Kelkheim (Taunus) (DE); Schwertfeger, Werner, Dr., D-6306 Langgöns (DE)

(56) Entgegenhaltungen:
- DE-A- 3 725 481
- US-A- 4 647 350
- CHEMICAL ABSTRACTS, Band 99, 7. November 1983, Columbus, Ohio, USA TOKUYAMA SODA CO., LTD: "Perfluoropolyethers" Seite 565, Zusammenfassung- Nr. 157 828t & Jpn. Kokai Tokkyo Koho JP 58 103 334

## Beschreibung

Die Erfindung betrifft ein Verfahren zur elektrolytischen Decarboxylierung einer Perfluorcarbonsäure der Formel (I) 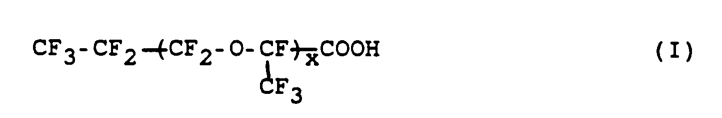
mit x = 1 bis 4
oder von löslichen Salzen dieser Säure oder eines Gemisches dieser Säure oder dieser Salze mit einer anderen Perfluorcarbonsäure der Formel (II) 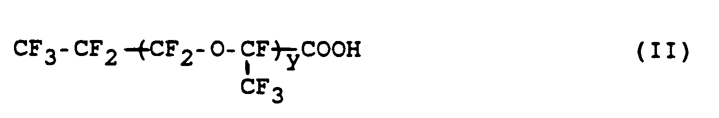
mit y = 0 bis 4
oder deren löslichen Salzen
und anschließende Dimerisierung der bei der Decarboxylierung entstandenen Radikale. Dabei werden Perfluorpolyether gebildet:
Falls man eine einzelne Perfluorcarbonsäure der Formel (I) oder eines ihrer löslichen Salze elektrolysiert, so wird nach folgendem Schema ein einzelner Perfluorpolyether gebildet: 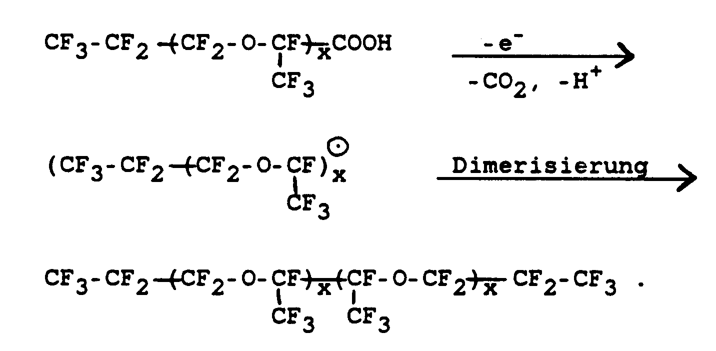
Wird dagegen eine Perfluorcarbonsäure der Formel (I) oder eines ihrer löslichen Salze im Gemisch mit einer anderen Perfluorcarbonsäure der Formel (II) 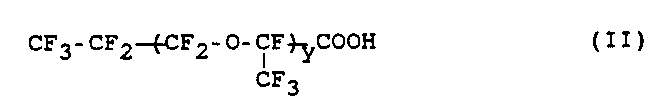
mit y = 0 bis 4
oder einem ihrer löslichen Salze elektrolysiert, so wird ein Gemisch der folgenden Perfluorpolyether gebildet:

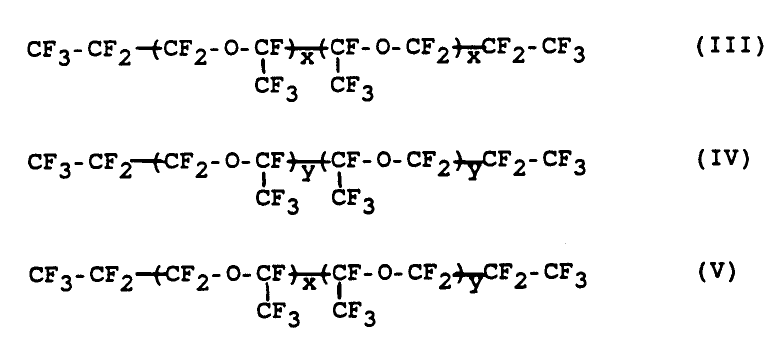

Derartige Perfluorpolyether sind Verbindungen, die aufgrund ihrer besonderen chemischen und physikalischen Eigenschaften zahlreiche Verwendungsgebiete haben. Die außerordentlich hohe chemische Beständigkeit gegen die meisten Stoffe, die auf dem perfluorierten Molekülgerüst der Perfluorpolyether beruht, erlaubt es beispielsweise, flüssige Perfluorpolyether lange Zeit ohne Zersetzung in ihrem Siedepunkt zu halten. Dabei können auch organische Stoffe und selbst geschmolzene Metalle zugegen sein, wie in Dampflötbädern für elektronische Bauteile ("vapour phase soldering"), ohne daß die Perfluorpolyether merklich angegriffen würden. Weiterhin können wegen dieser Inertheit flüssige Perfluorpolyether etwa zum Ausprüfen von elektronischen Komponenten verwendet werden. Dabei ist auch von Vorteil, daß diese Verbindungen in einem großen Temperaturintervall flüssig sind. Solche typische Ausprüfungen elektronischer Bauteile sind z.B. der "Thermal Shock Test", der "Gross Leak Test" und der "Burn In Test" (EP-A-0 203 348).

Weiterhin zeichnen sich derartige Perfluorpolyetherflüssigkeiten aus durch hohe elektrische Durchschlagsfestigkeit (> 150 kV·cm⁻¹ bei 20 °C), äußerst geringe elektrische Leitfähigkeit (< 10⁻⁹ S·cm⁻¹ bei 25 °C) und eine geringe Dielektrizitätskonstante (< 2 bei 25 °C). Sie können deshalb beispielsweise zum Kühlen von elektrischen Geräten unter Spannung benutzt werden.
Eine weitere besondere Eigenschaft ist das ausgezeichnete Sauerstofflösevermögen dieser Flüssigkeiten. Sie sind deshalb als Sauerstoffüberträger verwendbar, z.B. beim Einsatz als künstliches Blut, wie es in der japanischen Offenlegungsschrift Sho-58-103 334 vorgeschlagen wird.

Die genannten Verwendungszwecke machen es wünschenswert, eine rationelle technische Herstellungsmethode für die obigen Perfluorpolyether zu finden.

Aus der oben genannten japanischen Offenlegungsschrift Sho-58-103 334 ist bereits die Herstellung von symmetrischen Perfluorpolyethern der Formel (III) mit x = 1,2 und von unsymmetrischen Perfluorpolyethern der Formel (V) mit x =1 und y = 2 durch elektrolytische Decarboxylierung und anschließende Dimerisierung der entstandenen Radikale bekannt.
Diese Elektrolyse wird in polyalkoholischen Solventien durchgeführt. Als Beispiele für solche werden Ethylenglykol, Propylenglykol und Glyzerin genannt. Die Durchführung der Elektrolyse in diesen Lösemitteln wird als sehr günstig beschrieben, sowohl was die Ausbeute an Dimerisierungsprodukten, als auch was deren Isolierung aus dem Elektrolyt betrifft. Dagegen wird Methanol als Lösemittel für diese Elektrolyse ausdrücklich als ungeeignet bezeichnet und diese Aussage durch ein Vergleichsbeispiel belegt. Demzufolge soll die Elektrolyse der Perfluorcarbonsäuren 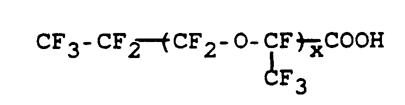
mit x = 1 oder 2 in Methanol oder Methanol-Wasser-Gemischen nur mit sehr niedrigen Ausbeuten der gewünschten Perfluorpolyether ablaufen, welche zudem nur schwierig von den in größerer Menge entstandenen Nebenprodukten zu isolieren seien.

Es wurde nun überraschenderweise gefunden, daß Methanol und Methanol-Wasser-Gemische als Elektrolytflüssigkeit für die obige Elektrolyse-Reaktion sehr gut geeignet sind. Dabei werden durchaus keine großen Mengen an Nebenprodukten gebildet; die Materialausbeute an den gewünschten Dimerisierungsprodukten ist vielmehr gut und in der Regel sogar besser als in den in der japanischen Offenlegungsschrift enthaltenen Ausführungsbeispielen. Außerdem wurde gefunden, daß die Abtrennung der Produkte in sehr einfacher Weise zu bewerkstelligen ist. Die Produkte bilden nämlich eine schwerere, im Elektrolyt unlösliche Phase und können deshalb am tiefsten Punkt einer Elektrolyseapparatur entnommen werden. Wird dem Elektrolyt während der Elektrolyse dann ständig Edukt zugeführt, so gelingt es auf einfache Weise, eine kontinuierlich arbeitende Elektrolyse durchzuführen, wie es in einigen der unten aufgeführten Ausführungsbeispiele beschrieben wird. Es ist bekannt, daß eine derartige kontinuierliche Methode in der Technik erhebliche Vorteile gegenüber einem absatzweise arbeitenden Verfahren bietet.

Gegenstand der vorliegenden Erfindung ist demgemäß ein Verfahren zur elektrolytischen Decarboxylierung einer Perfluorcarbonsäure der Formel (I) 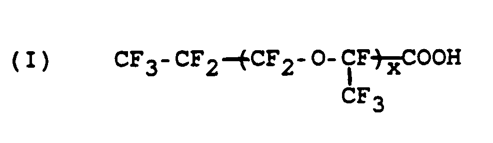
mit x = 1 bis 4
oder von löslichen Salzen dieser Säure oder eines Gemisches dieser Säure oder dieser Salze mit einer anderen Perfluorcarbonsäure der Formel (II) 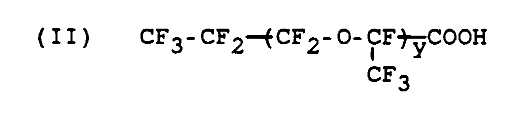
mit y = 0 bis 4
oder deren löslichen Salzen und anschließende Dimerisierung der bei der Decarboxylierung entstandenen Radikale, dadurch gekennzeichnet, daß man die Elektrolyse in Methanol oder einem Methanol-Wasser-Gemisch durchführt und die dabei entstandenen Perfluorpolyether isoliert.

Als Methanol-Wasser-Gemische setzt man im allgemeinen solche mit einem Wassergehalt von höchstens 90 Gew.-%, vorzugsweise höchstens 70 Gew.-% ein.

Die Elektrolyse kann im Prinzip in einem beliebigen zur Elektrolyse geeigneten Behälter durchgeführt werden, in dem sich Anode und Kathode gegenüberstehen. Bevorzugt sind jedoch Durchflußzellen, in denen der Elektrolyt durch einen Elektrodenspalt strömt. Eine Trennung in Anolyt- und Katholytraum durch eine Membran oder ein Diaphragma ist nicht erforderlich.
Als Elektroden können die zur Kolbe-Elektrolyse üblicherweise verwendeten Materialien zum Einsatz kommen. Als Anode werden Platin, mit Platin beschichtete Metalle und glasartiger Kohlenstoff bevorzugt; als Kathodenmaterial werden Elektrodengrafite und rostfreier Stahl bevorzugt.

Die Elektrolyse kann bei Temperaturen zwischen -30 °C und +60 °C durchgeführt werden, jedoch wird ein Bereich zwischen -10 °C und +25 °C bevorzugt.

Die Stromdichte ist, wie für Kolbe-Elektrolysen typisch, vorzugsweise möglichst hoch zu wählen. Ein technisch besonders geeigneter Bereich liegt zwischen 200 mA·cm⁻² und 500 mA·cm⁻².

Die Konzentration der umzusetzenden Säuren im Elektrolyt sollte vorzugsweise möglichst hoch gewählt werden, da dadurch der Anteil an Nebenprodukten minimiert wird. Die obere Grenze der Konzentration wird im wesentlichen bestimmt durch die Löslichkeit der Säuren im Elektrolyt.

Vorzugsweise setzt man lösliche Salze der Perfluorcarbonsäuren statt der freien Säuren ein, insbesondere die Natrium- oder Kalium-Salze.

### Ausführungsbeispiele

### Beispiel 1

Die Elektrolyse wurde durchgeführt in einer Umlaufapparatur, die aus Durchflußelektrolysezelle, Vorratsbehälter mit Kühlung und Umwälzpumpe bestand. Die Elektrolysezelle war bestückt mit einem platinierten Titanblech als Anode, sowie einer Elektrodengrafitplatte (®Diabon, Fa. Sigri) als Kathode mit jeweils 200 cm² Elektrodenfläche. 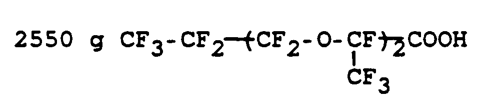
wurden in einem Gemisch von 2 l Methanol und 0,5 l Wasser, welches 10 g NaOH enthielt, gelöst und dann mit einer Stromstärke von 45 A solange elektrolysiert, bis 150 % der theoretisch erforderlichen Strommenge erreicht waren. In einer Absetzzone am Boden der Vorratsbehälters sammelten sich während dieser Elektrolyse 1914 g einer im Elektrolyt unlöslichen flüssigen Phase. Sie wurde abgetrennt, mit Natronlauge und Wasser gewaschen und dann destilliert. Danach wurden 1818 g (78,4 % d. Theorie) des Polyethers der Formel 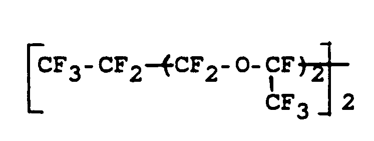
erhalten.

### Beispiel 2

In der in Beispiel 1 beschriebenen Elektrolyseapparatur wurden 2722 g CF₃-CF₂-CF₂-O-CF(CF₃)-COOH in einem Gemisch von 2,5 l Methanol und 2 l Wasser, welches 15 g NaOH enthielt, vorgelegt. Im Verlauf der Elektrolyse dieser Lösung wurden laufend weitere 2952 g obiger Säure in derjenigen stöchiometrischen Menge zugefügt, die der Menge an laufend erzeugtem und sich selbst separierendem Rohprodukt entsprach. Nach Zufuhr von 110 % der theoretisch erforderlichen Strommenge wurde 399 g Rohprodukt erhalten. Nach Waschen mit Natronlauge und Wasser und nach Destillation blieben 3776 g (77 % der theoretischen Ausbeute) des Polyethers der Formel

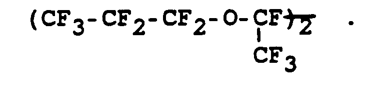

### Beispiel 3

In der in Beispiel 1 beschriebenen Elektrolyseapparatur wurden 2 kg eines äquimolaren Gemisches von 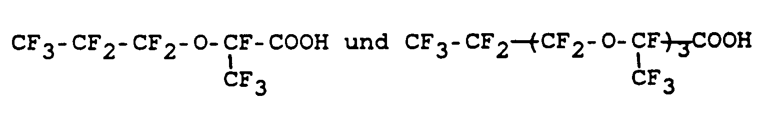
in 3,6 l Methanol und 0,9 l Wasser, welches 10 g NaOH enthielt, elektrolysiert. Im weiteren Verlauf der Elektrolyse wurden, wie in Beispiel 2 beschrieben, entsprechend der abgetrennten Rohproduktmenge, weitere 4 kg Eduktgemisch zugefügt. Nach Zufuhr von 138 % der theoretisch erforderlichen Strommenge wurden 5078 g Rohprodukt abgetrennt, die nach Waschen und Destillation folgende Menge an Produkten ergaben:

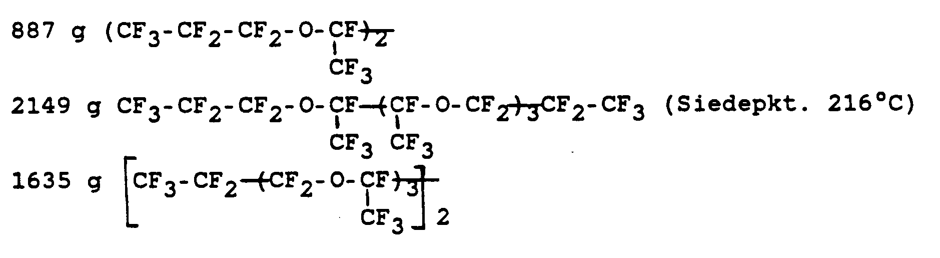

### Beispiel 4

In der in Beispiel 1 beschriebenen Elektrolyseapparatur wurden 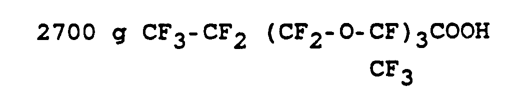
in einem Gemisch von 2 l Methanol und 0,5 l Wasser, das 8 g NaOH enthielt, solange elektrolysiert, bis 155 % der theoretisch erforderlichen Strommenge zugeführt waren. Das sich als schwerere Phase vom Elektrolyt separierende Rohprodukt wurde mit Natronlauge und Wasser gewaschen und dann destilliert. Danach wurden 2205 g (87,8 % der theoretischen Ausbeute) an 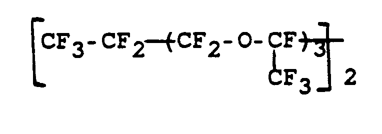
erhalten.

## Patentansprüche

1. Verfahren zur elektrolytischen Decarboxylierung einer Perfluorcarbonsäure der Formel (I) 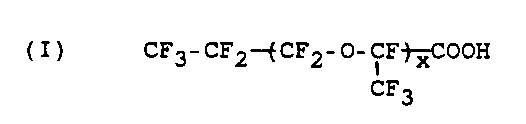 mit x = 1 bis 4
oder von löslichen Salzen dieser Säure oder eines Gemisches dieser Säure oder dieser Salze mit einer anderen Perfluorcarbonsäure der Formel (II) 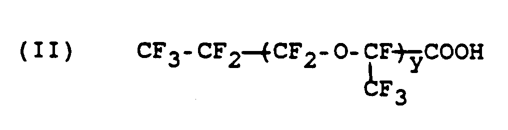 mit y = 0 bis 4
oder deren löslichen Salzen und anschließende Dimerisierung der bei der Decarboxylierung entstandenen Radikale, dadurch gekennzeichnet, daß man die Elektrolyse in Methanol oder einem Methanol-Wasser-Gemisch durchführt und die dabei entstandenen Perfluorpolyether isoliert.

2. Verfahren nach Anspruch 1, dadurch gekennzeichnet, daß man ein Methanol-Wasser-Gemisch mit einem Wassergehalt von höchstens 90 Gew.-% verwendet.

3. Verfahren nach Anspruch 1, dadurch gekennzeichnet, daß man ein Methanol-Wasser-Gemisch mit einem Wassergehalt von höchstens 70 Gew.-% verwendet.

## Claims

1. A process for the electrolytic decarboxylation of a perfluorocarboxylic acid of the formula (I): 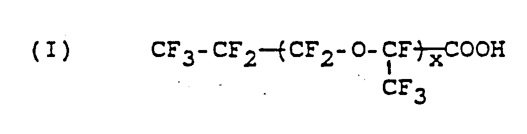 where x = 1 to 4,
or soluble salts of this acid, or a mixture of this acid or these salts with another perfluorocarboxylic acid of the formula (II): 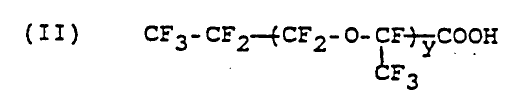 where y = 0 to 4,
or soluble salts thereof, and subsequent dimerization of the radicals resulting from decarboxylation, which comprises carrying out the electrolysis in methanol or a methanol/water mixture and isolating the resulting perfluoropolyethers.

2. The process as claimed in claim 1, wherein a methanol/water mixture with a water content of at most 90% by weight is used.

3. The process as claimed in claim 1, wherein a methanol/water mixture with a water content of at most 70% by weight is used.

## Revendications

1. Procédé pour décarboxyler électrolytiquement un acide perfluoro-carboxylique de la formule (I) : 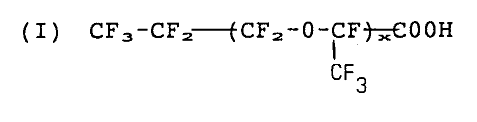 avec x = 1 à 4
ou des sels solubles de cet acide ou un mélange de cet acide ou de ces sels avec un autre acide perfluoro-carboxylique de la formule (II) : 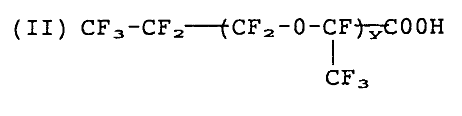 avec y = 0 à 4
ou des sels solubles de celui-ci, et dimériser ensuite les radicaux formés lors de la décarboxylation, procédé caractérisé en ce que l'on effectue l'électrolyse dans du méthanol ou dans un mélange eau-méthanol et on isole les perfluoro-polyéthers qui se sont alors formés.

2. Procédé selon la revendication 1, caractérisé en ce que l'on utilise un mélange eau-méthanol ayant une teneur en eau d'au plus 90 % en poids.

3. Procédé selon la revendication 1, caractérisé en ce que l'on utilise un mélange eau-méthanol ayant une teneur en eau d'au plus 70 % en poids.
